# FASCICULE DE BREVET EUROPEEN

(11) **EP 4 297 845 B1**
(45) Date de publication et mention de la délivrance du brevet: **26.11.2025**
(21) Numéro de dépôt: 22702201.9
(22) Date de dépôt: 20.01.2022
(51) Int. Cl.: A61N 1/05, A61B 5/293, A61B 5/00, A61B 5/291, A61B 5/25, H05K 1/02, H05K 1/11

(54) **SONDE D'EXPLORATION FONCTIONNELLE INTRACÉRÉBRALE MULTI-CONTACT**
MEHRKONTAKTSONDE FÜR INTRAZEREBRALE FUNKTIONELLE ERFORSCHUNG
MULTI-CONTACT INTRACEREBRAL FUNCTIONAL EXPLORATION PROBE

(30) Priorité: 27.01.2021 FR 2100760
(43) Date de publication de la demande: 03.01.2024
(73) Titulaire: NEXT NEUROTECH, 30000 Nîmes (FR)
(72) Inventeur: VALORGE, Yoann, 39700 RANCHOT (FR); MOUREAUX, Christophe, 25000 BESANÇON (FR); DAVID, Sébastien, 25000 BESANÇON (FR)
(74) Mandataire: Ipsilon
(86) Numéro de dépôt international: PCT/EP2022/051234
(87) Numéro de publication internationale: WO 2022/161849

(56) Documents cités:
- US-A1- 2014 303 703
- US-A1- 2016 144 165
- US-A1- 2018 289 949
- US-A1- 2019 175 905

## Description

### Domaine technique

La présente invention concerne le domaine médical. En particulier, l'invention concerne une sonde d'exploration fonctionnelle intracérébrale multi-contact ainsi qu'un procédé de fabrication d'une telle sonde. L'invention concerne également un dispositif d'exploration fonctionnelle intracérébrale, et/ou de stimulation et/ou de traitement par radio fréquence, multi-contact comportant une telle sonde.

### Technique antérieure

Afin de diagnostiquer ou de traiter certaines pathologies, comme par exemple une épilepsie pharmaco-résistante ou la maladie de parkinson, il est utile de se servir de sondes intracérébrales implantées, généralement provisoirement, dans l'organisme d'un patient.

De telles sondes peuvent être implantées dans le cerveau d'un patient pour enregistrer les activités électriques intracérébrales lors d'une stéréo-encéphalographie (SEEG) ou produire des stimulations électriques et, le cas échéant, identifier une anomalie puis éventuellement la traiter. Les sondes intracérébrales sont implantées selon un schéma d'implantation préalablement élaboré pour chaque patient en fonction des hypothèses sur l'origine de la pathologie.

Les sondes intracérébrales sont généralement des dispositifs de 15 à 100 cm de long et, en se plaçant du côté du chirurgien, peuvent être décomposées en trois parties:
- la partie « distale », c'est-à-dire à distance du chirurgien, destinée à être implantée dans l'organisme du patient,
- la partie « proximale », c'est-à-dire à proximité du chirurgien, destinée à relier la sonde à un appareil d'enregistrement ou de traitement et/ou un appareil de transmission de signal, et
- une partie intermédiaire reliant la partie distale et la partie proximale, cette partie intermédiaire étant généralement cylindrique.

La partie distale est composée d'une alternance de zones conductrices reliées par des pistes conductrices à un connecteur en partie proximale.

Pour fabriquer la partie distale de ces sondes, il est connu d'utiliser un tube cylindrique en matériau polymère thermoplastique sur lequel sont apposées des bagues métalliques.

Un autre procédé de fabrication connu consiste à juxtaposer et à solidariser, par soudage ou par collage, une succession de pièces cylindriques comportant respectivement un matériau polymère thermoplastique et un matériau métallique.

Dans les deux cas, le nombre de zones conductrices est limité par la solidité de l'assemblage de la sonde. En effet, afin de garantir la sécurité sanitaire de ces sondes, elles doivent être suffisamment solides afin de pouvoir être retirées sans laisser de corps étrangers dans le cerveau du patient. Le nombre de zones conductrices est ainsi limité par la solidité de l'assemblage de la sonde.

Des exemples de sondes intracérébrales multi-contact sont décrites par exemple dans les documents US2014/303703 A1 et US2019/175905 A1.

I1 existe ainsi un besoin de disposer d'une sonde d'exploration fonctionnelle intracérébrale multi-contact solide, fiable et pouvant comporter un grand nombre de zones conductrices.

### Exposé de l'invention

La présente invention parvient à répondre à ce besoin en tout ou partie grâce à, selon l'un de ses aspects, une sonde d'exploration fonctionnelle intracérébrale, et/ou de stimulation et/ou de traitement, notamment par radio fréquence, multi-contact telle que définie par la revendication 1.

Une telle sonde comporte :
- une partie distale de forme cylindrique comportant au moins un contact intracérébral, destinée à être implantée dans le cerveau d'un patient,
- une partie proximale de forme cylindrique comportant au moins un contact connecteur, destinée à être connectée à au moins un appareil d'enregistrement et/ou de stimulation et/ou traitement extérieur au corps du patient, et
- une partie de liaison de forme non cylindrique, notamment sensiblement plane, reliant la partie distale et la partie proximale.

La partie distale, la partie proximale et la partie de liaison comprennent un film multicouche comportant un substrat et au moins une couche conductrice déposée sur le substrat. Le substrat comporte au moins un matériau polymère et est réalisé de préférence d'une seule pièce. Ladite au moins une couche conductrice comporte au moins une piste de transmission et, dans ladite partie distale, ledit au moins un contact intracérébral ainsi que, dans la partie proximale, ledit au moins un contact connecteur, chaque piste de transmission étant reliée à un contact intracérébral en partie distale et à un contact connecteur en partie proximale.

Lorsque le substrat est réalisé d'une seule pièce, la partie distale, la partie proximale et la partie de liaison sont ainsi obtenues à partir d'un même substrat.

Ledit au moins un contact intracérébral est destiné à être en contact avec le cerveau d'un patient afin de capter un signal électrique caractéristique d'une activité cérébrale ou envoyer un signal électrique dans une zone du cerveau du patient pour effectuer un traitement.

La forme sensiblement plane de ladite partie de liaison lui procure une flexibilité importante, ce qui facilite sa manipulation pour le personnel médical et le confort pour le patient, notamment lorsque la sonde est implantée dans le cerveau d'un patient pendant plusieurs jours.

Par « cylindre », on entend une surface réglée dont les génératrices sont parallèles, c'est-à-dire une surface dans l'espace constituée de droites parallèles. Le cylindre est pourvu de deux ouvertures extrémales, chaque ouverture pouvant s'inscrire dans un plan parallèle de l'autre. La section du cylindre peut avoir différentes formes, comme par exemple circulaire, sensiblement circulaire, ovoïde, ovale, carrée, rectangulaire, étoilée, ou autre forme. La section des cylindres formant la partie distale et la partie proximale est de préférence sensiblement circulaire.

Par « au moins une couche conductrice », on entend au moins une couche de matériau avec une bonne conductivité électrique, par exemple une couche en matériau métallique, en graphite, ou en tout autre matériau avec une bonne conductivité électrique, en particulier biocompatible pour les zones susceptibles d'entrer en contact avec le patient. Ladite au moins une couche conductrice peut former uniquement des lignes formant les pistes de transmissions et, notamment aux extrémités de celles-ci, les contacts intracérébraux et connecteur.

La ou les couches conductrices peuvent comporter de l'or et/ou du platine et/ou du cuivre et/ou de l'iridium et/ou toute autre matériau conducteur biocompatible, de préférence du platine et/ou de l'or.

De préférence, la ou chaque couche conductrice présente une épaisseur comprise entre 1 µm et 1 000 µm environ, de préférence entre 5 µm et 15 µm environ.

La sonde d'exploration fonctionnelle intracérébrale multi-contact selon l'invention est fiable. En effet, étant donné que la partie distale de la sonde comporte un nombre limité de pièces fixées entre elles, notamment une seule pièce, le risque de déstructuration de la sonde est faible lors de son insertion ou de son extraction.

Le fait que la ou chaque piste de transmission soit reliée à un contact intracérébral et à un contact connecteur permet la transmission du ou des signaux captés par ledit contact intracérébral. Lorsqu'il y a plusieurs contacts intracérébraux, chacun d'entre eux est relié de préférence à une piste de transmission distincte qui lui est propre, qui lui est associée, chacun d'entre eux étant aussi relié de préférence à un contact connecteur distinct qui lui est propre, qui lui est associé.

La sonde peut comporter entre 1 et 60 contacts intracérébraux, notamment entre 2 et 60 contacts intracérébraux, notamment entre 2 et 20 contacts intracérébraux.

Dans un mode de réalisation, chaque contact intracérébral s'étend sur la totalité de la circonférence de la partie distale de la sonde.

En variante, au moins un contact intracérébral s'étend sur une partie seulement de la circonférence de la partie distale de la sonde.

Lorsque la sonde comporte plusieurs contacts intracérébraux, ils peuvent être à distance les uns des autres, les contacts intracérébraux adjacents deux à deux étant séparés d'une distance inter-contact qui peut être constante ou varier. A l'endroit de l'espace entre deux contacts intracérébraux adjacents, une couche isolante peut être présente.

Les contacts intracérébraux déposés peuvent présenter des longueurs identiques ou différentes. Par « longueur », on entend la longueur dudit au moins un contact intracérébral dans une direction parallèle à un axe longitudinal du substrat.

Ledit au moins un contact intracérébral peut comporter au moins deux parties séparées.

Le ou les contacts intracérébraux peut(vent) présenter une forme circulaire, avec des rayons identiques ou différents.

La partie distale peut comporter une extrémité proximale fermée, par exemple par un bouchon ou par le film multicouche lui-même.

Chaque contact intracérébral peut être relié à un unique contact connecteur par une unique piste de transmission. Le contact connecteur permet de faire le point de connexion avec l'appareil d'enregistrement et/ou de stimulation et/ou traitement extérieur au corps du patient pour la transmission des signaux électriques dans un sens et dans l'autre.

Ladite partie proximale et/ou ladite partie distale peut s'étendre selon un axe longitudinal. Un tel axe longitudinal peut être droit, courbe ou peut comporter des portions droites et des portions courbes. L'axe longitudinal des parties proximale et/ou distale peut être celui du substrat, notamment au moment de la formation du cylindre formant la partie proximale et/ou distale, mais l'orientation des parties distale et proximale peut bien entendu varier notamment lors de l'utilisation de la sonde, étant de préférence mobiles l'une par rapport à l'autre notamment à l'aide de la partie de liaison.

Il est préférable d'isoler du cerveau du patient ladite au moins une piste de transmission. Le film multicouche peut comporter, déposée sur le substrat, au moins une couche isolante en un matériau polymère, de préférence en un matériau polymère à cristaux liquides. Dans ce cas, ladite au moins une couche isolante peut recouvrir au moins partiellement ladite au moins une piste de transmission. Ladite au moins une couche isolante recouvre avantageusement en totalité ladite au moins une piste de transmission, à l'exception des extrémités de ladite au moins une piste de transmission en contact avec ledit au moins un contact intracérébral et avec ledit au moins un contact connecteur. De cette façon, ledit au moins un contact intracérébral et ladite au moins une piste de transmission sont reliés par une *via,* ou puits, dans ladite au moins une couche isolante, ladite *via* s'étendant de préférence transversalement, notamment orthogonalement, à ladite au moins une couche isolante. De même, ledit au moins un contact connecteur et ladite au moins une piste de transmission sont reliés par une *via,* ou puits, dans ladite au moins une couche isolante, ladite *via* s'étendant de préférence transversalement, notamment orthogonalement, à ladite au moins une couche isolante. En revanche, ladite au moins une couche isolante est prévue de préférence pour ne pas recouvrir ledit au moins un contact intracérébral, de telle sorte que celui-ci soit en contact avec le cerveau et puisse capter ou transmettre des signaux électriques.

Ladite au moins une couche isolante peut comporter un matériau polymère choisi dans le groupe constitué par les polymères à cristaux liquides, les polyamides, les silicones ou tout autre matériau polymère thermoplastique biocompatible, de préférence un matériau polymère à cristaux liquides.

La ou les couches isolantes sont par exemple réalisées dans le même matériau que le substrat.

La ou les couches isolantes peu(ven)t être déposée(s) puis fixée(s) sur le substrat par une compression et/ou un chauffage de la ou des couches isolantes.

Le cas échéant, la ou chaque couche isolante présente, de préférence, une épaisseur comprise entre 1 µm et 1 600 µm environ, de préférence entre 20 µm et 30 µm environ.

Le substrat comporte, de préférence, au moins un matériau polymère à cristaux liquides (PCL ou, en anglais, LCP pour Liquid Cristal polymer).

Un substrat comportant au moins un matériau polymère à cristaux liquides permet de conférer à la sonde une bonne fiabilité à long terme. Cela permet, par exemple, une insertion de la sonde dans le cerveau d'un patient pendant plusieurs jours, voire plusieurs mois, avec une bonne qualité de transmission de signaux électriques pendant toute cette durée.

De préférence, le substrat présente une épaisseur comprise entre 1 µm et 160 µm environ, de préférence entre 25 µm et 90 µm environ.

La partie distale présente une forme cylindrique, donc un cylindre définissant une cavité intérieure. La cavité intérieure formée par le cylindre de la partie distale peut être au moins partiellement remplie d'au moins une colle ou d'un matériau polymère ou d'un matériau composite, notamment un silicone chargé de particules métalliques. Le remplissage peut être homogène ou hétérogène.

La sonde peut comporter un plot distal. Dans ce cas, la partie distale peut comporter une extrémité distale fermée par le plot distal. Un tel plot distal peut être réalisé dans au moins un matériau conducteur, notamment métallique. Dans ce cas, le plot distal, lorsqu'il est conducteur, peut former un contact intracérébral indépendant du ou des autres contacts intracérébraux. Toujours dans ce cas, la sonde comporte avantageusement une piste de transmission conductrice en contact avec le plot distal, lorsque conducteur. La sonde peut en variante comporter un fil de transmission relié au plot distal, par exemple relié à un contact connecteur sur la partie proximale.

Lorsque la cavité intérieure formée par le cylindre de la partie distale est au moins partiellement remplie avec du silicone chargé de particules métalliques et lorsque la sonde comporte un plot distal conducteur, le silicone chargé de particules métalliques peut permettre d'établir une connexion électrique entre le plot distal conducteur et un fil de transmission ou une piste de transmission conductrice en contact avec le silicone chargé.

La partie distale peut comporter au moins un capteur de température, notamment formé par ladite au moins une couche conductrice. Dans ce cas, la sonde d'exploration fonctionnelle intracérébrale multi-contact permet d'obtenir une ou plusieurs données thermiques sur le cerveau du patient. Le ou chaque capteur de température peut être un capteur de température à résistance, par exemple à résistance de platine. Le capteur de température peut comporter un thermocouple.

La présente description a encore pour objet, indépendamment et/ou en combinaison avec ce qui précède, selon un autre de ses aspects, une sonde d'exploration fonctionnelle intracérébrale, et/ou de stimulation et/ou de traitement, notamment par radio fréquence, multi-contact comportant une partie distale destinée à être implantée dans le cerveau d'un patient comportant une partie distale de forme cylindrique comportant au moins un contact intracérébral et au moins un capteur de température, destinée à être implantée dans le cerveau d'un patient.

La partie distale comprend un film multicouche comportant un substrat et au moins une couche conductrice déposée sur le substrat. Le substrat comporte au moins un matériau polymère, étant de préférence réalisé d'une seule pièce. Ladite au moins une couche conductrice comporte au moins une piste de transmission, ledit au moins un contact intracérébral et ledit au moins un capteur de température, chaque piste de transmission étant reliée à un contact intracérébral.

La présente description a encore pour objet, indépendamment et/ou en combinaison avec ce qui précède, selon un autre de ses aspects, une sonde d'exploration fonctionnelle intracérébrale, et/ou de stimulation et/ou de traitement, notamment par radio fréquence, multi-contact comportant une partie distale destinée à être implantée dans le cerveau d'un patient de forme cylindrique comportant au moins un contact intracérébral.

La partie distale comprend un film multicouche comportant un substrat et au moins une couche conductrice déposée sur le substrat. Le substrat comporte au moins un matériau polymère, étant de préférence réalisé d'une seule pièce. Ladite au moins une couche conductrice comporte au moins une piste de transmission et ledit au moins un contact intracérébral, chaque piste de transmission étant reliée à un contact intracérébral.

Selon cet aspect, la partie distale comprend une extrémité distale fermée par un plot distal.

La présente description a encore pour objet, indépendamment et/ou en combinaison avec ce qui précède, selon un autre de ses aspects, une sonde d'exploration fonctionnelle intracérébrale, et/ou de stimulation et/ou de traitement, notamment par radio fréquence, multi-contact comportant une partie distale destinée à être implantée dans le cerveau d'un patient, de forme cylindrique, comportant au moins un contact intracérébral.

La partie distale comprend un film multicouche comportant un substrat et au moins une couche conductrice déposée sur le substrat. Le substrat comporte au moins un matériau polymère, étant réalisé de préférence d'une seule pièce. Ladite au moins une couche conductrice comporte au moins une piste de transmission et ledit au moins un contact intracérébral, chaque piste de transmission étant reliée à un contact intracérébral.

Selon cet aspect de de la présente description, le film multicouche comporte deux bords latéraux dans la partie distale, les deux bords latéraux étant en contact en bord à bord.

### Procédé de fabrication d'une sonde d'exploration fonctionnelle intracérébrale multi-contact

L'invention a encore pour objet un procédé de fabrication d'une sonde d'exploration fonctionnelle intracérébrale, et/ou de stimulation et/ou de traitement, notamment par radio fréquence, multi-contact telle que défini par la revendication 8.

Un tel procédé comporte les étapes suivantes :
a) Etape a : former un film multicouche en déposant à plat, sur au moins une partie du substrat, au moins une couche conductrice formant au moins un contact intracérébral, au moins un contact connecteur et au moins une piste de transmission, chaque piste de transmission étant reliée à un contact intracérébral et à un contact connecteur,
b) Etape b : former un cylindre s'étendant selon un axe longitudinal à partir d'au moins une première partie du film multicouche destinée à former ladite partie distale de la sonde destinée à être implantée dans le cerveau d'un patient, afin d'obtenir cette dernière,
c) Etape c : former un cylindre s'étendant selon un axe longitudinal à partir d'au moins une deuxième partie du film multicouche destinée à former ladite partie proximale de la sonde destinée à être connectée à au moins un appareil d'enregistrement et/ou de stimulation et/ou traitement extérieur au corps du patient, afin d'obtenir cette dernière.

Par « en déposant à plat », on entend que, au cours de l'étape a, lors du dépôt de la ou des couches conductrices, le substrat a une forme sensiblement plane.

Les étapes b et c peuvent être réalisées simultanément.

Un unique substrat, réalisé de préférence d'une seule pièce, est utilisé pour former ladite partie distale, ladite partie de liaison, et ladite partie proximale. Le procédé permet ainsi de fabriquer une sonde d'exploration fonctionnelle intracérébrale multi-contact avec un nombre limité de pièces fixées entre elles, notamment une unique pièce, ce qui limite les risques de déstructuration de celle-ci lors de l'insertion ou de l'extraction de la partie distale de la sonde dans le cerveau d'un patient et limite son encombrement.

Ainsi, la partie distale, la partie proximale et la partie de liaison constituent de préférence une seule et unique pièce.

Les parties distales et proximales sont avantageusement identiques hormis la géométrie des contacts intracérébraux et des contacts connecteur.

De préférence, le substrat est réalisé en au moins un matériau polymère à cristaux liquides (PCL). En effet, les matériaux polymères à cristaux liquides ne sont pas endommagés ni dissous par des solvants organiques utilisés en micro-fabrication, comme par exemple un alcool, une acétone, une résine photosensible, un révélateur/dissolvant de résines photosensibles ou un agent de gravure acide pour les métaux. Cette résistance aux solvants permet de déposer ladite au moins une couche conductrice avec une grande précision, par exemple à l'aide d'un procédé de revêtement par centrifugation, un procédé de métallisation, un procédé de photolithographie ou un procédé de gravure sèche ou humide.

Ainsi, l'utilisation d'un substrat en au moins un matériau polymère à cristaux liquides facilite et améliore le dépôt de ladite au moins une couche conductrice.

L'étape a peut consister à former une ou plusieurs couches, notamment une ou plusieurs couches conductrices, sur le substrat. Pour ce faire, l'étape a peut consister à réaliser un empilement (ou une superposition) de différentes couches, notamment de différentes couches conductrices.

Ledit au moins un contact connecteur est de préférence formé sur ladite deuxième partie.

L'étape a peut, après dépôt de ladite au moins une couche conductrice sur le substrat, comporter une compression et/ou un chauffage de ladite au moins une couche conductrice sur le substrat de manière à fixer ladite au moins une couche conductrice sur le substrat.

Ladite au moins une couche conductrice est de préférence produite séparément puis déposée sur le substrat.

Le procédé peut comporter, notamment avant l'étape b, une étape de finition de ladite au moins une couche conductrice, comme par exemple un décapage et/ou une gravure sèche ou humide.

Il est préférable voire nécessaire d'isoler ladite au moins une piste de transmission du cerveau du patient. Le procédé peut ainsi comporter l'étape consistant à déposer, avant l'étape b, au moins une couche isolante en un matériau polymère sur le substrat, ladite au moins une couche isolante recouvrant au moins partiellement ladite au moins une piste de transmission. De cette façon, chaque signal capté par le ou chaque contact intracérébral peut être transmis *via* la piste de transmission qui lui est associée sans être dégradé ou perturbé par un éventuel contact électrique entre la piste de transmission et le cerveau du patient.

De préférence, le substrat à plat est allongé selon un axe longitudinal.

L'étape a peut être mise en œuvre de manière à ce que ledit au moins un contact intracérébral présente une surface de largeur transversale comprise entre 0,1 mm et 10 mm environ, de préférence égale à 2 mm environ et de longueur longitudinale comprise entre 0,1 mm et 10 mm environ, de préférence égale à 2 mm environ.

Par « largeur transversale », on entend la largeur dudit au moins un contact intracérébral dans une direction transversale à l'axe longitudinal du substrat.

Par « longueur longitudinale », on entend la longueur dudit au moins un contact intracérébral dans une direction parallèle à l'axe longitudinal du substrat.

Ledit au moins un contact intracérébral peut s'étendre sur toute la largeur transversale du substrat ou, en variante, sur une partie seulement de celle-ci.

Lorsque plusieurs contacts intracérébraux sont déposés sur le substrat, ils peuvent être déposés à distance les uns des autres, les contacts intracérébraux adjacents étant séparés d'une distance inter-contact qui peut être constante ou varier. A l'endroit de l'espace entre deux contacts intracérébraux, il est possible de déposer une couche isolante.

Ledit au moins un contact intracérébral peut comporter au moins deux parties séparées et s'étendre à partir d'une extrémité latérale du substrat.

Le ou les contacts intracérébraux peut(vent) être déposé(s) de manière à présenter une forme circulaire, avec des rayons identiques ou différents.

Le procédé peut comporter, avant l'étape b de mise en forme, une étape de découpage du substrat. Ce découpage peut être réalisé avant ou après l'étape a.

Dans un mode de réalisation particulier, au cours de l'étape a, au moins un capteur de température est déposé sur le substrat par collage, soudage ou empilement, de préférence sur ladite première partie du film. Dans ce cas, la sonde d'exploration fonctionnelle intracérébrale multi-contact permet d'obtenir une ou plusieurs données thermiques sur le cerveau du patient. Le ou chaque capteur de température peut être un capteur de température à résistance, par exemple à résistance de platine. Un tel capteur peut être formé par au moins une couche conductrice déposée au cours de l'étape a. Le capteur de température peut comporter un thermocouple.

Ladite première partie et/ou ladite deuxième partie du film peut(vent) comporter deux bords latéraux. Dans ce cas, l'étape b peut comporter un enroulement au moins partiel de ladite première partie et/ou ladite deuxième partie du film sur elle-même.

Dans un mode de réalisation, ladite première partie et/ou ladite deuxième partie du film comportant deux bords latéraux. l'étape b et/ou l'étape c comporte(nt) un enroulement au moins partiel de ladite première partie et/ou ladite deuxième partie du film sur elle-même, notamment sous la forme d'une spirale, de manière à au moins partiellement superposer lesdits bords latéraux.

Par « deux bords latéraux », on entend inclure, pour chaque bord, la partie d'extrémité latérale du film multicouche et également une partie de la surface du film multicouche à proximité de cette extrémité latérale.

Lesdits bords latéraux peuvent être fixés entre eux par collage et/ou par soudage.

Dans un autre mode de réalisation, ladite première partie du film et/ou ladite deuxième partie du film comportant deux bords latéraux, l'étape b et/ou c comporte(nt) un enroulement au moins partiel de ladite première partie du film et/ou ladite deuxième partie du film de manière à mettre en contact en bord à bord lesdits bords latéraux, c'est-à-dire sans superposition desdits bords.

Le substrat peut comporter sur au moins un desdits bords latéraux au moins une fenêtre, l'étape a pouvant être réalisée de manière à déposer ladite au moins une couche conductrice en dehors de ladite au moins une fenêtre. La présence de fenêtre(s) peut permettre de faciliter la fixation desdits bords entre eux, la ou chaque fenêtre augmentant la longueur de fixation entre les deux bords latéraux.

Dans l'un ou l'autre de ces modes de réalisation, l'enroulement de ladite première partie et/ou ladite deuxième partie du film peut être réalisé en insérant successivement ladite première partie et/ou ladite deuxième partie du film dans au moins un cône tronqué, le diamètre de la section circulaire équivalente de ladite première partie et/ou ladite deuxième partie du film étant diminué après l'insertion successive dans chaque cône tronqué.

Dans un autre mode de réalisation, le substrat présentant dans son épaisseur une cavité intérieure fermée latéralement mais ouverte à au moins une extrémité longitudinale, l'étape b consiste à remplir ladite cavité intérieure du substrat avec au moins un matériau notamment biocompatible.

Le procédé peut, après l'étape b comportant notamment un enroulement de ladite première partie du film, comporter un remplissage au moins partiel d'une cavité intérieure formée par le cylindre de la partie distale. Un tel remplissage peut être réalisé par l'injection d'au moins une colle ou d'un matériau polymère, d'un matériau composite, notamment un silicone chargé en particules métalliques.

Lorsque le procédé comporte, après l'enroulement au moins partiel de ladite première partie du film, un remplissage au moins partiel d'une cavité intérieure formée par le cylindre de la partie distale, ce remplissage est notamment fait à proximité desdits bords latéraux, étant de préférence réalisé par l'injection d'au moins une colle ou d'un matériau polymère ou d'un matériau composite, notamment un silicone. Ce remplissage peut être homogène ou hétérogène.

Le procédé comporte avantageusement, notamment pendant ou après l'étape b, l'étape consistant à insérer un plot distal, notamment comportant au moins un matériau conducteur, par exemple métallique, dans une extrémité distale du film multicouche.

Un tel plot distal peut être fixé par collage, soudage, moulage, surmoulage et/ou fixation mécanique, par exemple par insertion à force. Un tel plot distal peut dépasser longitudinalement de l'extrémité distale du film multicouche et former l'extrémité distale de la partie distale. Avantageusement, lorsqu'il est réalisé au moins partiellement en métal, le plot distal forme un contact intracérébral indépendant dudit au moins un contact intracérébral déposé sur le substrat au cours de l'étape a, c'est-à-dire que le plot distal n'est pas en contact avec ledit au moins un contact intracérébral ni avec ladite au moins une piste de transmission. Dans ce cas, l'étape a peut comporter le dépôt sur le substrat d'au moins une couche conductrice formant au moins une piste de transmission distale, ladite au moins une piste de transmission distale étant destinée à être en contact avec ledit plot distal, après l'insertion de celui-ci dans ladite extrémité distale.

Ladite au moins une couche conductrice peut être déposée au cours de l'étape a pour former au moins un contact connecteur, de préférence sur ladite deuxième partie, relié à ladite au moins une piste de transmission distale.

Un bouchon peut être inséré dans une extrémité proximale de la partie distale, par exemple par remplissage, par moulage, par surmoulage et/ou par fixation mécanique, notamment par insertion à force, par collage ou soudage. Le bouchon peut ne pas dépasser, notamment longitudinalement, de l'extrémité proximale.

La présente description a encore pour objet, indépendamment ou en combinaison avec ce qui précède, un procédé de fabrication d'une sonde d'exploration fonctionnelle intracérébrale, et/ou de stimulation et/ou de traitement, notamment par radio fréquence, multi-contact comportant une partie distale destinée à être implantée dans le cerveau d'un patient, le procédé comportant les étapes suivantes :
a) Etape a : former un film multicouche comportant une première partie destinée à former ladite partie distale en déposant à plat, sur au moins une partie d'un substrat, au moins une couche conductrice formant au moins un contact intracérébral et au moins une piste de transmission, chaque piste de transmission étant reliée à un contact intracérébral, ladite première partie du film comportant deux bords latéraux,
b) Etape b : former un cylindre s'étendant selon un axe longitudinal à partir de ladite première partie du film afin d'obtenir la partie distale, l'étape b comportant un enroulement au moins partiel de ladite première partie du film sur elle-même, l'enroulement de ladite première partie du film étant réalisé en insérant successivement ladite première partie dans au moins deux cônes tronqués, le diamètre de la section circulaire équivalente de ladite première partie du film étant diminué après l'insertion successive dans chaque cône tronqué.

Le procédé peut comporter la fabrication d'une partie proximale de la sonde d'exploration fonctionnelle intracérébrale multi-contact. Dans ce cas, ledit film multicouche peut comporter une deuxième partie destinée à former ladite partie proximale, ladite au moins une couche conductrice pouvant former au moins un contact connecteur sur cette deuxième partie, ledit au moins un contact connecteur étant relié à une piste de transmission.

Le procédé peut comporter l'étape consistant à former un cylindre s'étendant selon un axe longitudinal à partir de ladite deuxième partie du film afin d'obtenir la partie proximale, cette étape comportant un enroulement au moins partiel de ladite deuxième partie du film sur elle-même, l'enroulement de ladite deuxième partie du film étant réalisé en insérant successivement ladite deuxième partie du film dans au moins deux cônes tronqués, le diamètre de la section circulaire équivalente de ladite deuxième partie du film étant diminué après l'insertion successive dans chaque cône tronqué.

### Dispositif d'exploration fonctionnelle intracérébrale multi-contact

L'invention a encore pour objet dispositif d'exploration fonctionnelle intracérébrale, et/ou de stimulation et/ou de traitement, notamment par radio fréquence, multi-contact tel que défini par la revendication 15.

Un tel dispositif comporte au moins une sonde d'exploration fonctionnelle intracérébrale multi-contact telle que définie précédemment et au moins un appareil d'enregistrement et/ou de stimulation et/ou de traitement du patient connecté à ladite au moins une sonde.

Le dispositif peut être relié à un appareil de traitement de données tiers, apte à traiter les signaux captés par le ou les contacts intracérébraux et transmis par la ou les pistes de transmission associées.

Le ou les contacts connecteur présents sur la partie proximale permettent de transmettre des signaux électriques depuis la sonde vers l'appareil d'enregistrement et/ou de traitement et/ou de stimulation du patient et/ou vers l'appareil de traitement de données, et vice versa.

Un tel dispositif peut permettre de réaliser un diagnostic de l'activité cérébrale du cerveau, par exemple une stéréo-encéphalographie (SEEG) et/ou un traitement d'une ou plusieurs zones du cerveau.

Un tel dispositif peut permettre de recueillir des données sur l'activité neuronale d'un patient.

Le dispositif peut permettre d'appliquer une stimulation électrique dans le cerveau d'un patient notamment à proximité dudit au moins un contact intracérébral. Cette stimulation peut être réalisée avec un signal électrique périodique, par exemple avec une fréquence comprise entre 1 et 1000 Hz environ, notamment avec une intensité comprise entre 0,1 et 20 mA environ.

Le dispositif peut permettre un traitement par thermo-coagulation à l'aide d'un signal électrique de haute-fréquence envoyé dans le cerveau d'un patient, notamment à proximité dudit au moins un contact intracérébral, par exemple à une fréquence comprise entre 400 kHz et 600 kHz environ, notamment avec une puissance comprise entre 0,0001 W et 10 W environ.

### Procédé d'exploration fonctionnelle intracérébrale ou de traitement intracérébral

L'invention trouve par exemple son application dans un procédé non revendiqué d'exploration fonctionnelle intracérébrale ou de stimulation et/ou de traitement intracérébral comportant les étapes suivantes :
Etape x : insérer au moins une sonde d'exploration fonctionnelle intracérébrale, et/ou de stimulation et/ou de traitement, notamment par radio fréquence, multi-contact tel que définie plus haut dans le cerveau d'un patient,
Etape y : connecter ladite au moins une sonde à au moins un appareil d'enregistrement et/ou de stimulation et/ou de traitement,
Etape z : mesurer une activité cérébrale nerveuse à l'aide du ou des contacts intracérébraux et/ou réaliser une stimulation électrique dans le cerveau du patient et/ou réaliser un traitement électrique dans le cerveau du patient.

### Brève description des dessins

L'invention pourra être mieux comprise à la lecture de la description détaillée qui va suivre, d'exemples de mise en œuvre non limitatifs de celle-ci, et à l'examen du dessin annexé, sur lequel :
[Fig 1] la figure 1 illustre de manière schématique et partielle, en perspective, un exemple de sonde d'exploration fonctionnelle intracérébrale, et/ou de stimulation et/ou de traitement, notamment par radio fréquence, multi-contact selon l'invention,
[Fig 2] la figure 2 illustre, de manière schématique et partielle, en coupe transversale, la partie du film multicouche formant la partie distale de la figure 1,
[Fig 3] la figure 3 illustre, de manière schématique, en perspective, un exemple de partie distale de la sonde selon l'invention,
[Fig 4] la figure 4 illustre, de manière schématique, en perspective, un autre exemple de partie distale de la sonde selon l'invention,
[Fig 5] la figure 5 illustre, de manière schématique, en perspective, un autre exemple de partie distale de la sonde selon l'invention,
[Fig 6] la figure 6 illustre, de manière schématique, en perspective, un autre exemple de partie distale de la sonde selon l'invention,
[Fig 7] la figure 7 illustre, de manière schématique, en perspective, un autre exemple de partie distale de la sonde selon l'invention,
[Fig 8] la figure 8 illustre, de manière schématique, en perspective, l'extrémité distale d'une partie distale d'un exemple de sonde selon l'invention,
[Fig 9] la figure 9 illustre, de manière schématique, en perspective, l'extrémité proximale d'une partie distale d'un exemple de sonde selon l'invention,
[Fig 10] la figure 10 illustre, de manière schématique, en perspective, un exemple de partie proximale de la sonde selon l'invention,
[Fig 11] la figure 11 illustre, de manière schématique, en perspective, un autre exemple de partie proximale de la sonde selon l'invention,
[Fig 12] la figure 12 est un schéma bloc d'un exemple de procédé de fabrication d'une sonde selon l'invention,
[Fig 13] la figure 13 illustre, de manière schématique, en vue du dessus, un exemple de film multicouche destiné à former une sonde selon l'invention,
[Fig 14] la figure 14 est une vue schématique de dessus d'un exemple de partie du film multicouche destinée à former la partie distale,
[Fig 15] la figure 15 est une vue similaire à la figure 14 d'un autre exemple de partie du film multicouche destinée à former la partie distale,
[Fig 16] la figure 16 est une vue similaire à la figure 14 d'un autre exemple de partie du film multicouche destinée à former la partie distale comportant deux capteurs de température,
[Fig 17] la figure 17 illustre isolément, de manière schématique en vue de dessus, l'un des capteurs de température de l'exemple de la figure 16,
[Fig 18] la figure 18 représente un schéma illustrant la connexion du capteur de température de la figure 16,
[Fig 19] la figure 19 illustre, de manière schématique, en perspective, différentes sous-étapes d'un exemple de mise en œuvre de l'étape b du procédé selon l'invention,
[Fig 20] la figure 20 illustre, de manière schématique, le remplissage de la cavité du cylindre formée par le film après l'étape b du procédé selon l'invention,
[Fig 21] la figure 21 illustre, de manière schématique, en perspective, un exemple de partie du film multicouche destinée à former la partie distale de la sonde selon l'invention,
[Fig 22] la figure 22 illustre, de manière schématique, en perspective, la partie distale formée à partir de la partie de film multicouche de la figure 21,
[Fig 23] la figure 23 illustre, de manière schématique, en vue de dessus, un autre exemple de film multicouche destiné à former la sonde selon l'invention,
[Fig 24] la figure 24 est une vue en coupe transversale schématique de la partie distale réalisée à partir du film multicouche de la figure 23 , et
[Fig 25] la figure 25 illustre de manière schématique, un dispositif d'exploration selon l'invention comportant plusieurs sondes selon l'invention, lors de son utilisation.

### Description détaillée

Dans la suite de la description, les éléments identiques ou de fonctions identiques portent le même signe de référence. A des fins de concision de la présente description, ils ne sont pas décrits en regard de chacune des figures, seules les différences entre les modes de réalisation étant décrites.

Sur les figures, les proportions réelles n'ont pas toujours été respectées, dans un souci de clarté.

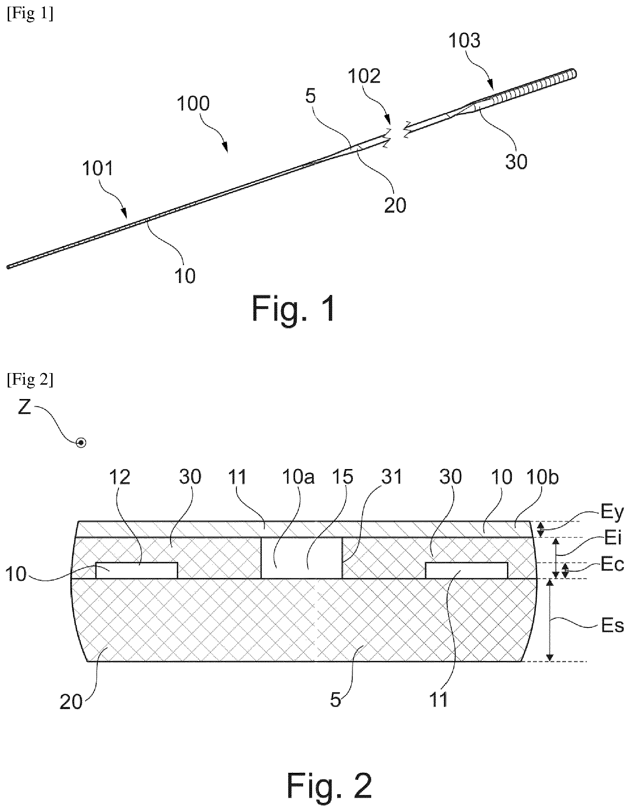

On a illustré à la figure 1 un exemple de sonde 100 d'exploration fonctionnelle intracérébrale, et/ou de stimulation et/ou de traitement, notamment par radio fréquence, multi-contact selon l'invention. La sonde 100 comporte une partie distale 101, mais également une partie proximale 103 et une partie de liaison 102 pour relier entre elles les parties distale 101 et proximale 103.

La partie distale 101 présente une forme cylindrique. Elle est destinée à être implantée dans le cerveau d'un patient.

La partie proximale 103 présente une forme cylindrique et est destinée à être connectée à au moins un appareil d'enregistrement et/ou de stimulation et/ou de traitement extérieur au corps du patient, non représenté sur cette figure.

La partie de liaison 102 présente une forme sensiblement plane.

La partie distale 101, la partie proximale 103 et la partie de liaison 102 sont formées par un film multicouche 5 comportant un substrat 20 et au moins une couche conductrice 10 déposée sur le substrat 20.

Le substrat 20 est, dans cet exemple, réalisé d'une seule pièce et comporte au moins un matériau polymère à cristaux liquides.

Dans cet exemple, le film multicouche 5 comporte également une couche isolante 30.

La couche conductrice 10, comme visible sur la figure 2, forme sur la partie distale 101 plusieurs contacts intracérébraux 11 et plusieurs pistes de transmission 12. Dans cet exemple, la couche isolante 30 recouvre les pistes de transmission 12 afin qu'elles ne puissent pas être en contact avec le cerveau du patient. Sur la figure 2, une partie seulement des pistes de transmission 12 sont représentées, pour des raisons de clarté du dessin. Chaque contact intracérébral 11 est dans cet exemple associé à une piste de transmission 12.

Dans l'exemple illustré, comme visible sur la figure 2, le film multicouche 5 dans la partie distale 101 comporte, outre le substrat 20, une première couche conductrice 10a déposée directement sur le substrat 20 et formant les pistes de transmission 12. Cette première couche conductrice 10a présente une épaisseur Ec de 10 µm et une surépaisseur Ei de 25 µm aux extrémités 15 des pistes de transmission 12.

Le film multicouche 5 comporte encore la couche isolante 30, dont l'épaisseur est Ei, également déposée sur le substrat 20 et recouvre la totalité du substrat 20 non recouvert par les pistes de transmission 12. à l'exception des extrémités 15 des pistes de transmission 12. La couche isolante 30 épouse le relief formé au niveau des pistes de transmission 12. La ou les couches isolantes 30 comportent, dans cet exemple, un matériau polymère à cristaux liquides, par exemple le même matériau que le substrat 20.

Le film multicouche 5 comporte une deuxième couche conductrice 10b d'épaisseur Ey de 10 µm, déposée sur la couche isolante 30 et formant les contacts intracérébraux 11, comme visible sur la figure 2.

Chaque piste de transmission 12 est en contact par son extrémité 15 à un contact intracérébral 11. De cette façon, les contacts intracérébraux 11 et les pistes de transmission 12 sont reliés par une *via* 31 dans la couche isolante 30.

Comme cela sera décrit plus en détails ci-après, il est à noter que le film multicouche 5 comporte, dans la partie proximale 103 de la sonde 100, le substrat 20, au moins une couche conductrice 10 formant les pistes de transmission 12 ainsi que des contacts connecteur 77 aux extrémités des pistes de transmission 12. Les pistes de transmission 12 sont recouvertes d'au moins une couche isolante 30. Les contacts connecteur 77 permettent de mettre en contact électrique la sonde 100 et l'appareil d'enregistrement et/ou de stimulation et/ou de traitement extérieur.

Enfin, dans la partie de liaison 102 de la sonde 100, le film multicouche 5 comporte le substrat 20, au moins une couche conductrice formant les pistes de transmission 12 et au moins une couche isolante 30 recouvrant les pistes de transmission 12.

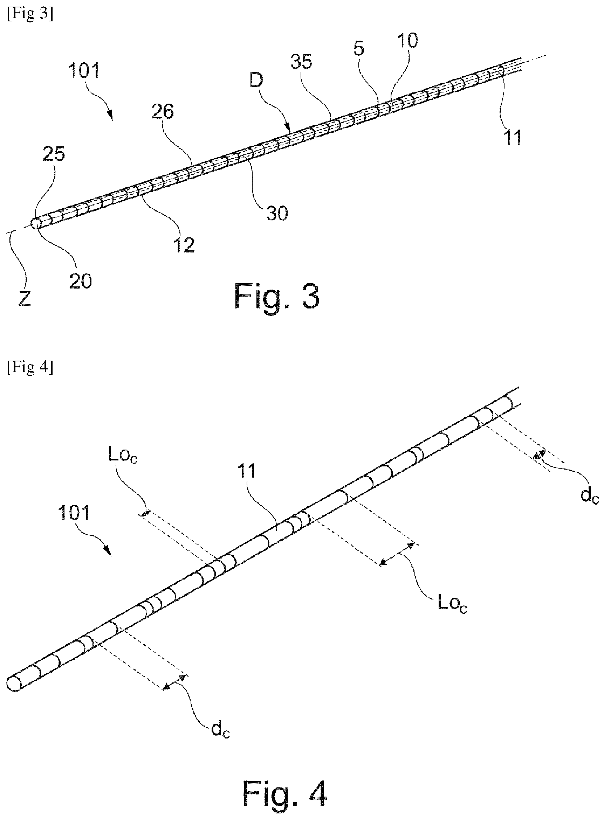

On voit sur la figure 3 le cylindre formé par la partie distale 101, définissant une cavité intérieure 25, qui présente une section circulaire de diamètre D extérieur de 0,7 mm environ. Dans cet exemple, chaque contact intracérébral 11 s'étend sur la totalité de la circonférence du cylindre.

Par ailleurs, dans cet exemple, la partie distale 101 comporte un capteur de température 35 formé par la couche conductrice 10.

On a illustré sur les figures 4 à 7 différents exemples de partie distale 101 d'une sonde 100. Sur ces figures, les pistes de transmission 12 n'ont pas été représentées, pour des raisons de clarté du dessin.

Dans l'exemple de la figure 4, les contacts intracérébraux 11 présentent des longueurs Lo_{c} différentes et sont espacés entre eux de distances d_{c} différentes, comme visible.

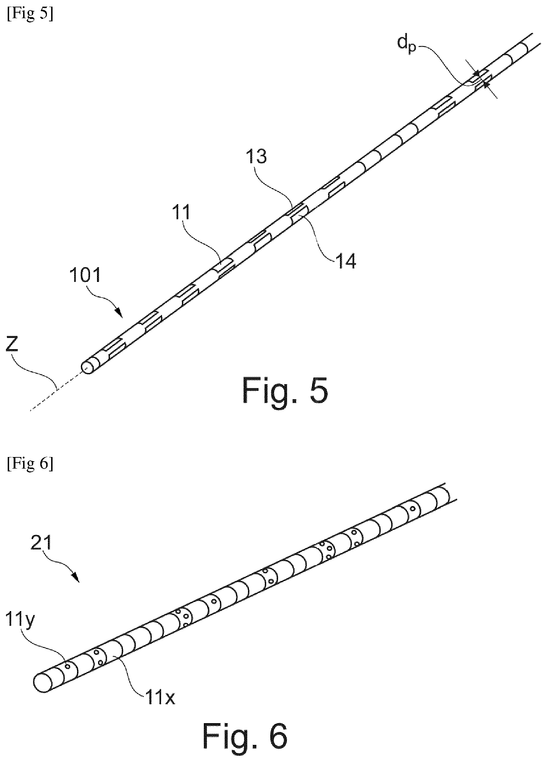

Dans l'exemple de la figure 5, les contacts intracérébraux 11 s'étendent sur une partie seulement de la circonférence du cylindre formé par la partie distale 101. Chaque contact intracérébral 11 qui s'étend transversalement à l'axe Z comporte deux parties 13 et 14 séparées l'une de l'autre par un espace de largeur dₚ. Toujours dans cet exemple, les espaces de largeur dₚ entre les parties 13 et 14 de plusieurs contacts intracérébraux 11 disposés à la suite les uns des autres ne sont pas alignés entre eux selon l'axe Z, mais sont décalés.

Dans l'exemple de la figure 6, la partie distale 101 comporte des contacts intracérébraux 11x s'étendant sur la totalité de la circonférence du cylindre formé par la partie distale 101 et des contacts intracérébraux 11y de forme circulaire.

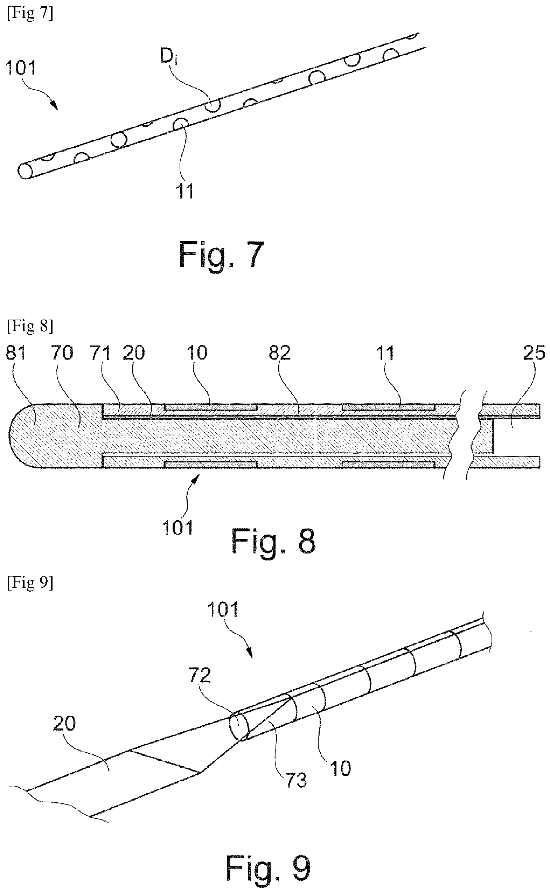

Dans l'exemple de la figure 7, la partie distale 101 comporte des contacts intracérébraux 11 de forme circulaire de diamètre Dᵢ égal à 2 mm environ. Les contacts intracérébraux 11 sont par ailleurs disposés en quinconce sur le film multicouche 5.

L'utilisation de contacts intracérébraux 11 de forme circulaire permet de réaliser des sondes 100 capables de réaliser une mesure ou impulsion directionnelle.

Dans l'exemple de réalisation illustré sur la figure 8, la sonde 100 comporte un plot distal 70, métallique dans cet exemple, inséré dans la cavité intérieure 25 à partir de l'extrémité distale 71 et fixé par collage ou par soudage ou par fixation mécanique. Le plot distal 70 forme alors un contact intracérébral 81 indépendant des autres contacts intracérébraux 11. Dans cet exemple, le plot distal 70 est en contact avec une piste de transmission 82 distale spécifique, déposée sur le substrat 20.

En variante ou en complément, la cavité intérieure 25 peut être partiellement remplie avec un silicone chargé de particules métalliques permettant de constituer et/ou d'établir une connexion électrique entre le plot distal 70 et un fil de transmission ou une piste de transmission 12 en contact avec le silicone chargé.

Dans le mode de réalisation de la figure 9, la partie distale 101 de la sonde 100 comporte un bouchon 72 qui a été inséré dans la cavité intérieure 25 au niveau de l'extrémité proximale 73 de la partie distale 101. Ce bouchon 72 est par exemple réalisé en silicone.

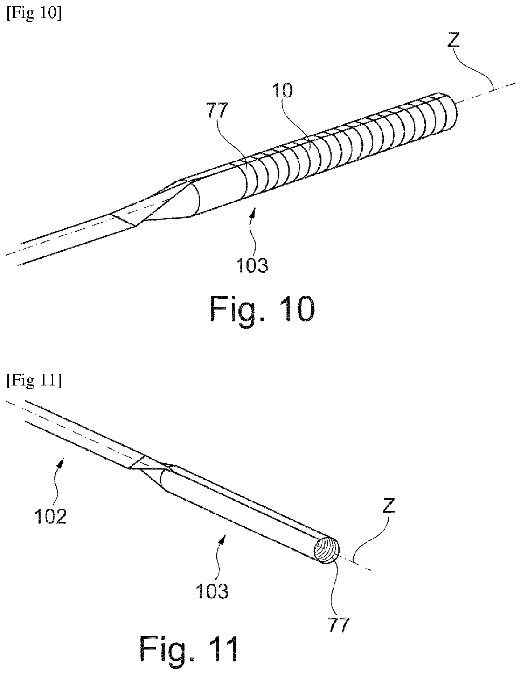

Les figures 10 et 11 illustrent la partie proximale 103 de la sonde 100. La partie proximale 103 de la sonde 100 s'étend selon l'axe longitudinal Z et comporte des contacts connecteur 77 formés par la couche conductrice 10, chacun d'eux étant relié à un unique contact intracérébral 11 présent sur la partie distale 101 par une unique piste de transmission 12 présente sur la partie proximale 103, la partie de liaison 102 et la partie distale 101.

Dans l'exemple de la figure 10, les contacts connecteur 77 de la partie proximale 103 sont disposés sur la surface extérieure du cylindre formé par la partie proximale 103.

En variante, dans l'exemple de la figure 11, les contacts connecteur 77 de la partie proximale 103 sont disposés sur la surface intérieure du cylindre formé par la partie proximale 103.

La partie de liaison 102 comporte des pistes de transmission 12 prolongeant les pistes de transmission 12 des parties distale 101 et proximale 103. Chaque contact intracérébral 11 est connecté à un unique contact connecteur 77 par une unique piste de transmission 12 parcourant partiellement la partie distale 101, la partie de liaison 102 et partiellement la partie proximale 103.

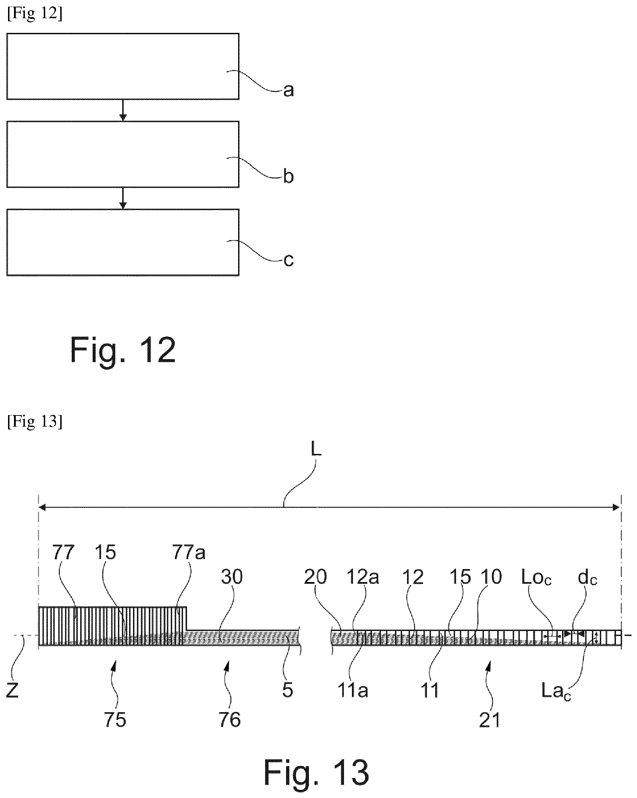

On a illustré sur la figure 12 un exemple de procédé de fabrication d'une sonde 100 d'exploration fonctionnelle intracérébrale multi-contact selon l'invention, comportant trois étapes a, b et c.

Dans la première étape a, et comme visible sur les figures 2 et 13, pour réaliser le film multicouche 5, au moins une, dans cet exemple plusieurs couches conductrices 10, dans cet exemple en or avec un revêtement en platine et en iridium, sont déposées à plat sur un substrat 20 réalisé en un matériau polymère à cristaux liquides (PCL).

Le substrat 20 est, dans cet exemple, découpé avant l'étape a et présente une longueur L égale à 1000 mm environ. Comme visible sur la figure 13, le substrat 20 est allongé, à plat, selon un axe longitudinal Z. Le substrat 20 présente une première partie 21 destinée à former la partie distale 101 de la sonde 100 d'exploration fonctionnelle intracérébrale multi-contact, une partie 76 destinée à former la partie de liaison 102 et une deuxième partie 75 destinée à former la partie proximale 103 de la sonde 100. Le substrat 20 présente dans cet exemple, transversalement à l'axe Z, une épaisseur Es égale à 50 µm, comme visible sur la figure 2.

Dans cet exemple, les différentes couches conductrices 10 sont déposées afin de former plusieurs contacts intracérébraux 11 dans la première partie 21, plusieurs contacts connecteur 77 dans la deuxième partie 75 et plusieurs pistes de transmission 12. Chaque contact intracérébral 11 est dans cet exemple associé à une piste de transmission 12 et à un contact connecteur 77.

Dans l'exemple illustré, le dépôt des couches conductrices 10 se fait de la manière décrite en regard de la figure 2. Les couches conductrices 10 et les couches isolantes 30 sont déposées par empilement sur le substrat 20 puis comprimées et chauffées.

Chaque contact intracérébral 11 est relié à un unique contact connecteur 77 par une unique piste de transmission 12. Par exemple, le contact intracérébral 11a est relié au contact connecteur 77a par la piste de transmission 12a.

Dans la deuxième étape b du procédé selon l'invention, on forme un cylindre s'étendant selon un axe longitudinal Z à partir d'une première partie 21 du film multicouche 5 destinée à former ladite partie distale 101 de la sonde 100 afin d'obtenir cette dernière.

Dans la troisième étape c du procédé selon l'invention, on forme un cylindre s'étendant selon l'axe longitudinal Z à partir d'une deuxième partie 75 du film multicouche 5 destinée à former la partie proximale 103 de la sonde 100 afin d'obtenir cette dernière.

Dans l'exemple illustré sur la figure 13, les contacts intracérébraux 11 présentent une largeur La_{c} de 2,5 mm s'étendant sur la totalité de la largeur du substrat 20 transversalement à l'axe Z et une longueur Lo_{c} de 2 mm, mesurée parallèlement à l'axe Z. Ils sont identiques entre eux. Les pistes de transmission 12 ont une largeur de 50 µm. Les contacts intracérébraux 11 sont espacés entre eux de manière régulière d'une distance d_{c} égale à 1,5 mm environ.

Toujours dans cet exemple, les contacts connecteur 77 sont déposés sur une largeur supérieure à la longueur La_{c} des contacts intracérébraux 11. Ils sont espacés de manière régulière et présentent une longueur constante mesurée parallèlement à l'axe Z.

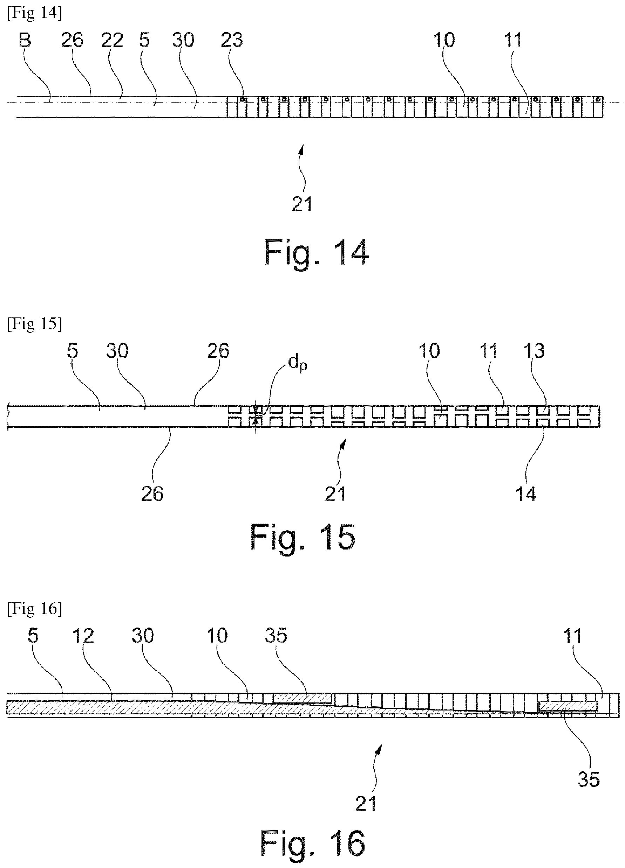

On a illustré sur les figures 14 à 16 différents exemples de première partie 21 du film multicouche 5 destinée à former la partie distale 101 d'une sonde 100 après l'étape a et avant l'étape b. Sur ces figures, les pistes de transmission 12 n'ont pas toujours été représentées, pour des raisons de clarté du dessin.

Sur la figure 14, la partie 21 comporte dix-huit contacts intracérébraux 11 de mêmes dimensions. Le substrat 20 comporte des fenêtres 23, traversantes, sur un bord latéral 22. Le bord latéral 22 s'étend d'une extrémité latérale 26 du substrat 20 jusqu'à une partie du substrat 20 à proximité de cette extrémité latérale 26, dans cet exemple jusqu'à la ligne B.

L'étape a est, dans cet exemple de la figure 14, réalisée de manière à déposer les couches conductrices 10 et les couches isolantes 30 en dehors des fenêtres 23. Le film multicouche 5 comporte alors lui-même des fenêtres 23. L'utilisation de fenêtres 23 peut permettre de faciliter la fixation des bords latéraux 22 entre eux, comme décrit par la suite.

Dans l'exemple de la figure 15, les contacts intracérébraux 11 ne s'étendent pas sur la totalité de la largeur du substrat 20, transversalement à l'axe Z, mais sur une partie seulement de celle-ci. Chaque contact intracérébral 11 qui s'étend transversalement à l'axe Z comporte deux parties 13 et 14 séparées l'une de l'autre par un espace de largeur dₚ. Chaque partie 13 ou 14 s'étend à partir d'une extrémité latérale 26 du substrat 20. Les espaces de largeur dₚ entre les parties 13 et 14 de plusieurs contacts intracérébraux disposés à la suite les uns des autres ne sont pas alignés entre eux selon l'axe Z dans cet exemple, mais sont décalés.

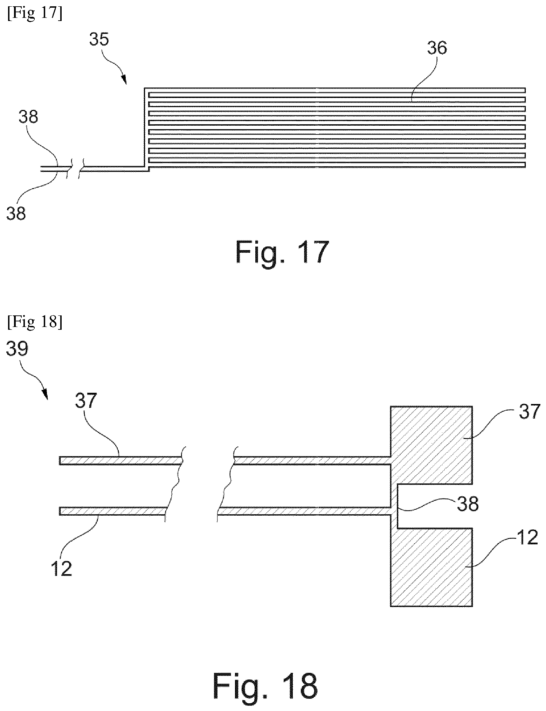

Dans l'exemple illustré sur la figure 16, on a formé, sur la première partie 21 du film multicouche 5, outre les contacts intracérébraux 11 et les pistes de transmission 12, deux capteurs de température 35 à résistance dont un agrandissement est illustré sur la figure 17.

Chaque capteur de température 35 comporte une longue piste 36, sous la forme d'un zig-zag, reliée à deux pistes 37 et 38. La piste 38 est reliée à une piste de transmission 12 reliée à un contact intracérébral 11, comme illustré sur la figure 18.

La résistance du circuit 39 entre la piste 37 et la piste de transmission 12 reliée à la piste 38 varie en fonction de la température.

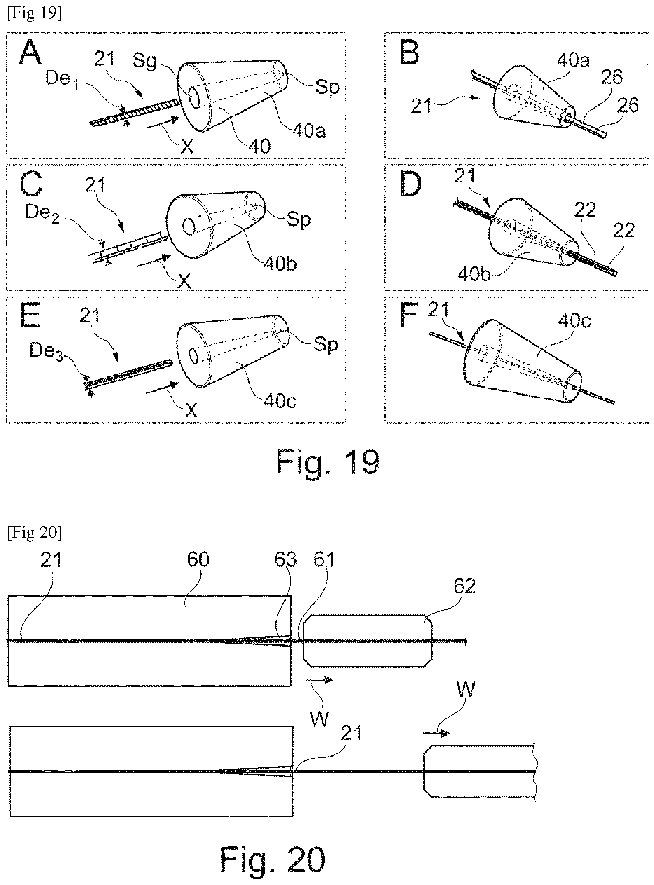

Un exemple de mise en œuvre de l'étape b a été illustré sur la figure 19. Dans cet exemple, l'étape b consiste à réaliser un enroulement de la première partie 21 à l'aide de trois cônes tronqués 40.

Dans ce mode de réalisation, on cherche à rapprocher en bord à bord les extrémités latérales 26 des bords latéraux 22.

Avant l'insertion du film multicouche 5 dans le premier cône 40a (vue A), le film multicouche 5 est à plat. Le diamètre De₁ de la section circulaire équivalente de la première partie 21 du film multicouche 5 est représenté en pointillé. Le film multicouche 5 est inséré dans le cône tronqué 40a, selon la direction représentée par la flèche X, au travers de la plus grande section Sg jusqu'à la plus petite section Sp.

Pendant l'insertion, la première partie 21 du film multicouche 5 se courbe pour former un arc de cercle, comme visible sur la vue B. Le diamètre De₂ de la section circulaire équivalente formé après l'insertion dans le cône tronqué 40a est inférieur au diamètre De₁ avant insertion.

La première partie 21 du film multicouche 5 est ensuite retirée du cône tronqué 40a pour être insérée selon la direction X dans la plus grande section Sg d'un cône tronqué 40b comportant une plus petite section Sg inférieure à la plus petite section Sg du cône tronqué 40a, comme visible sur la vue C.

Après l'insertion dans le cône tronqué 40b, la première partie 21 s'enroule davantage autour d'elle-même, comme visible sur la vue D, et le diamètre De₃ de sa section circulaire équivalente est inférieur au diamètre De₂.

La première partie 21 du film multicouche 5 est ensuite retirée du cône tronqué 40b pour être insérée selon la direction X dans la plus grande section Sg d'un cône tronqué 40c comportant une plus petite section Sg inférieure à la plus petite section Sg du cône tronqué 40b, comme visible sur la vue E.

Après l'insertion dans le cône tronqué 40c, la première partie 21 s'enroule davantage autour d'elle-même, comme visible sur la vue F, et les deux bords latéraux 22 entrent en contact.

Le cylindre ainsi formé après l'étape b, présente une section circulaire, forme la cavité intérieure 25 et présente, dans cet exemple, un diamètre D extérieur de 0,8 mm environ. Chaque contact intracérébral 11 s'étend sur la totalité de la circonférence du cylindre formé dans cet exemple.

La première partie 21 du film multicouche 5 forme ainsi la partie distale 101 de la sonde 100 d'exploration fonctionnelle intracérébrale.

Il est, de même, possible de réaliser l'étape c par un enroulement de la deuxième partie 75 du film multicouche 5 destinée à former la partie proximale 103 de la sonde 100 à l'aide de cônes tronqués de manière analogue à ce qui vient d'être décrit.

Afin de maintenir la forme cylindrique de la première partie 21 du film multicouche 5, on peut remplir la cavité intérieure 25, dans cet exemple avec du silicone. Le procédé de remplissage est illustré sur la figure 20 et décrit ci-après.

La première partie 21 du film multicouche 5 est insérée dans une cavité 63 ouverte sur une extrémité d'un moule 60. Un mandrin 61 est inséré dans la cavité formée par la première partie 21 du film multicouche 5. Le mandrin 61 est relié à une réserve de silicone 62, qui peut également servir de moyen de préhension. Au fur et à mesure de l'injection du silicone, le mandrin 61 est progressivement retiré de la cavité intérieure 25 dans la direction W.

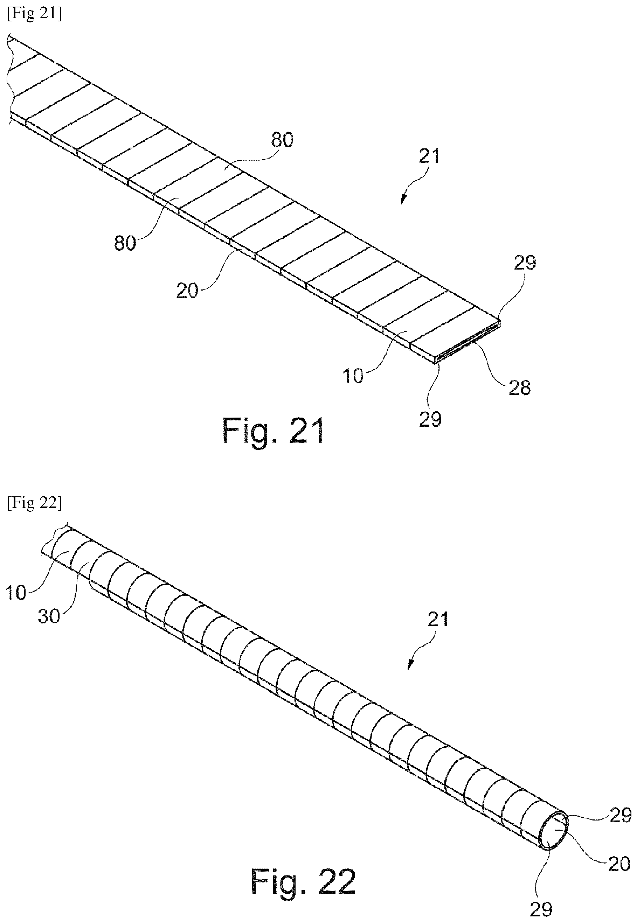

D'autres exemples de mise en œuvre du procédé selon l'invention sont décrits en regard des figures 21 à 24.

Dans le mode de réalisation illustré sur les figures 21 et 22, dans la première partie 21 du film multicouche 5, le substrat 20 présente dans son épaisseur une cavité intérieure 28 fermée latéralement mais ouverte à au moins une extrémité longitudinale, dans l'exemple illustré aux deux extrémités longitudinales. Dans ce cas, le substrat 20 peut être fabriqué, avant l'étape a, en superposant deux couches 29 de matériau polymère à cristaux liquides puis en soudant leurs bords 80 transversaux.

Dans cet exemple, l'étape b est réalisée par remplissage de la cavité intérieure 28 du substrat 20 avec au moins un matériau biocompatible, de manière à former la partie distale 101, cylindrique.

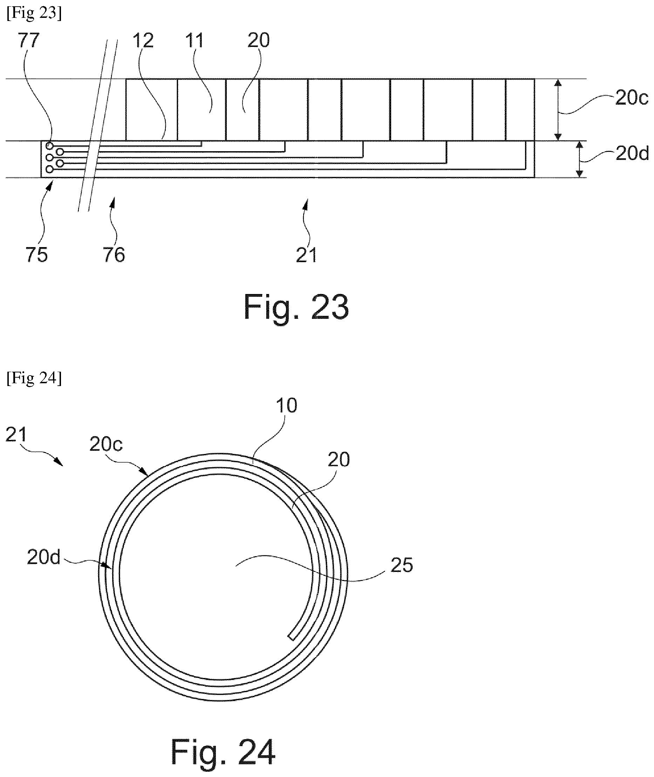

Dans le mode de réalisation illustré sur les figures 23 et 24, les contacts intracérébraux 11 sont déposés au cours de l'étape a dans une partie 20c du substrat 20 et les pistes de transmission 12 et les contacts connecteur 77 dans une partie 20d du substrat 20, les parties 20c et 20d étant adjacentes latéralement mais distinctes. Chaque contact intracérébral 11 est lié à un unique contact connecteur 77 par une unique piste de transmission 12. Les pistes de transmission 12 ne se croisent pas.

Toujours dans cet exemple, l'étape b du procédé est réalisée en enroulant le film multicouche 5 autour de lui-même de manière à ce que la partie 20c forme la périphérie extérieure du cylindre formé, comme visible sur la figure 24. La partie 20d est disposée à l'intérieur de la cavité intérieure 25 formée par le cylindre.

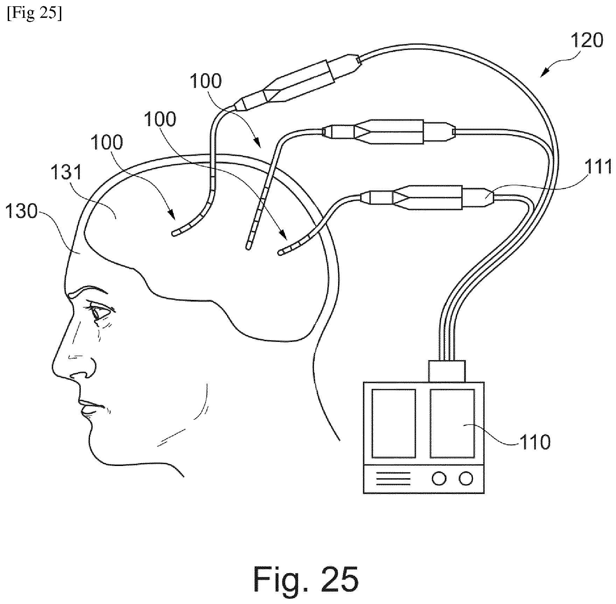

La sonde 100 peut être connectée à un appareil d'enregistrement et/ou de de stimulation et/ou traitement du patient 110 à l'aide d'un connecteur 111 pour former un dispositif 120 d'exploration fonctionnelle intracérébrale, et/ou de stimulation et/ou de traitement, notamment par radio fréquence, multi-contact, comme illustré sur la figure 25.

L'appareil d'enregistrement et/ou de stimulation et/ou de traitement du patient 110 permet également de faire du traitement de données reçues au niveau des contacts connecteur 77, transmises par les pistes de transmission 12 en provenance des contacts intracérébraux 11.

Un tel dispositif 120 peut permettre de réaliser une exploration fonctionnelle intracérébrale ou un traitement ou une stimulation intracérébral(e), comme illustré sur la figure 25, comportant les étapes suivantes :
Etape x : insérer au moins une sonde 100 d'exploration fonctionnelle intracérébrale multi-contact telle que décrite plus haut, dans cet exemple trois sondes 100, dans le cerveau 131 d'un patient 130,
Etape y : connecter les sondes 100 à au moins un appareil d'enregistrement et/ou de stimulation et/ou de traitement 110, et
Etape z : mesurer une activité électrique cérébrale et/ou réaliser une stimulation électrique dans le cerveau 131 du patient 130 et/ou réaliser un traitement électrique dans le cerveau 131 du patient 130.

L'invention n'est pas limitée aux exemples qui viennent d'être décrits.

En particulier, le procédé peut comporter une étape de finition de la couche conductrice, comme par exemple une gravure.

Le nombre de contacts intracérébraux peut être différent, par exemple être compris entre 2 et 60.

La couche conductrice 10 peut être déposée par un autre procédé, en particulier par un procédé de dépôt de couches minces.

Le film multicouche 5 peut être enroulé sur lui-même d'une manière différente, par exemple en utilisant plus ou moins de cônes tronqués.

Le plot distal 70 peut être connecté à un appareil d'enregistrement et/ou de stimulation et/ou de traitement du patient par un fil de connexion.

Les différentes couches déposées sur le substrat 20 peuvent avoir des épaisseurs différentes.

Il est possible de déposer plusieurs couches conductrices et/ ou isolantes, par exemple entre 2 et 10 couches conductrices et/ou isolantes, sur le substrat 20.

Les couches isolantes 30 peuvent comporter un autre matériau polymère, en particulier un autre matériau polymère biocompatible, comme du polyamide.

Dans une variante non illustrée, l'enroulement de la première partie 21 du film multicouche 5 est réalisé de manière à superposer partiellement les bords latéraux 22, par exemple à l'aide de cônes tronqués.

Dans le cas où l'enroulement de la première partie 21 du film multicouche 5 est réalisé de manière à superposer partiellement les bords latéraux 22, on peut fixer les bords latéraux 22 entre eux par collage ou par soudage.

La présente invention est définie par les revendications ci-après.

## Revendications

1. Sonde (100) d'exploration fonctionnelle intracérébrale, et/ou de stimulation et/ou de traitement par radio fréquence, multi-contact comportant :
• une partie distale (101) de forme cylindrique comportant au moins un contact intracérébral (11), destinée à être implantée dans le cerveau (131) d'un patient,
• une partie proximale (103) de forme cylindrique comportant au moins un contact connecteur (77), destinée à être connectée à au moins un appareil d'enregistrement et/ou de stimulation et/ou de traitement (110) extérieur au corps du patient, et
• une partie de liaison (102) de forme non cylindrique reliant la partie distale (101) et la partie proximale (103),
la partie distale (101), la partie proximale (103) et la partie de liaison (102) comprenant un film multicouche (5) comportant un substrat (20) et au moins une couche conductrice (10) déposée sur le substrat (20), le substrat (20) comportant au moins un matériau polymère, ladite au moins une couche conductrice (10) comportant au moins une piste de transmission (12) et, dans la partie distale (101), ledit au moins un contact intracérébral (11) ainsi que, dans la partie proximale (103), ledit au moins un contact connecteur (77), chaque piste de transmission (12) étant reliée à un contact intracérébral (11) en partie distale (103) et à un contact connecteur (77) en partie proximale (103).

2. Sonde (100) selon la revendication 1, le film multicouche (5) comportant, déposée sur le substrat (20), au moins une couche isolante (30) en un matériau polymère, de préférence en un matériau polymère à cristaux liquides, ladite au moins une couche isolante (30) recouvrant au moins partiellement ladite au moins une piste de transmission (12).

3. Sonde (100) selon l'une des revendications précédentes, comportant entre 1 et 60 contacts intracérébraux (11), notamment entre 2 et 60 contacts intracérébraux (11).

4. Sonde (100) selon l'une quelconque des revendications précédentes, le substrat (20) étant réalisé en au moins un matériau polymère à cristaux liquides (PCL).

5. Sonde (100) selon l'une quelconque des revendications précédentes, la cavité intérieure (25) formée par le cylindre de la partie distale (101) étant au moins partiellement remplie d'au moins une colle ou d'un matériau polymère ou d'un matériau composite, notamment un silicone chargé de particules métalliques.

6. Sonde (100) selon l'une quelconque des revendications précédentes, la partie distale (101) comportant au moins un capteur de température (35), notamment formé par ladite au moins une couche conductrice (10).

7. Sonde (100) selon l'une quelconque des revendications précédentes, la partie distale (101) comportant une extrémité distale (71) fermée par un plot distal (70) et/ou la partie distale (101) comportant une extrémité proximale (73) fermée par un bouchon (72) ou par le film multicouche (5) lui-même.

8. Procédé de fabrication d'une sonde (100) d'exploration fonctionnelle intracérébrale, et/ou de stimulation et/ou de traitement par radio fréquence, multi-contact selon l'une quelconque des revendications précédentes, le procédé comportant les étapes suivantes :
a) Etape a : former un film multicouche (5) en déposant à plat, sur au moins une partie du substrat (20), au moins une couche conductrice (10) formant au moins un contact intracérébral (11), au moins un contact connecteur (77) et au moins une piste de transmission (12), chaque piste de transmission (12) étant reliée à un contact intracérébral (11) et à un contact connecteur (77),
b) Etape b : former un cylindre s'étendant selon un axe longitudinal (Z) à partir d'au moins une première partie (21) du film multicouche (5) destinée à former ladite partie distale (101) de la sonde (100) destinée à être implantée dans le cerveau (131) d'un patient afin d'obtenir cette dernière,
c) Etape c : former un cylindre s'étendant selon un axe longitudinal (Z) à partir d'au moins une deuxième partie (75) du film multicouche (5) destinée à former ladite partie proximale (101) de la sonde (100) destinée à être connectée à au moins un appareil d'enregistrement et/ou de stimulation et/ou de traitement (110) extérieur au patient, afin d'obtenir cette dernière.

9. Procédé selon la revendication précédente, l'étape a, après dépôt de ladite au moins une couche conductrice (10) sur le substrat (20), comportant une compression et/ou un chauffage de ladite au moins une couche conductrice (10) sur le substrat (20) de manière à fixer ladite au moins une couche conductrice (10) sur le substrat (20).

10. Procédé selon l'une des revendications 8 à 9, ladite première partie (21) et/ou ladite deuxième partie (75) du film multicouche (5) comportant deux bords latéraux (22), l'étape b et/ou l'étape c comportant un enroulement au moins partiel de ladite première partie (21) et/ou ladite deuxième partie (75) du film multicouche (5) sur elle-même(s) de manière à au moins partiellement superposer lesdits bords latéraux (22), lesdits bords latéraux (22) étant de préférence fixés entre eux par collage et/ou par soudage.

11. Procédé selon l'une des revendications 8 à 9, ladite première partie (21) et/ou ladite deuxième partie (75) du film multicouche (5) comportant deux bords latéraux (22), l'étape b et/ou c comportant un enroulement au moins partiel de ladite première partie (21) et/ou ladite deuxième partie (75) du film multicouche (5) de manière à mettre en contact en bord à bord lesdits bords latéraux (22).

12. Procédé selon la revendication 10 ou 11, dans lequel le substrat (20) comporte sur au moins un desdits bords latéraux (22) au moins une fenêtre (23), l'étape a étant réalisée de manière à déposer ladite au moins une couche conductrice (10) en dehors de ladite au moins une fenêtre (23).

13. Procédé selon l'une quelconque des revendications 10 à 12, dans lequel l'enroulement de ladite première partie (21) et/ou ladite deuxième partie (75) du film multicouche (5) est(sont) réalisé(s) en insérant successivement ladite première partie (21) du et/ou ladite deuxième partie (75) du film multicouche (5) dans au moins un cône tronqué (40 ; 40a, 40b, 40c), le diamètre (dₑ) de la section circulaire équivalente de ladite première partie (21) et/ou ladite deuxième partie (75) du film multicouche (5) étant diminué après l'insertion successive dans chaque cône tronqué (40 ; 40a, 40b, 40c).

14. Procédé selon l'une des revendications 8 et 9, dans lequel le substrat (20) présente dans son épaisseur une cavité intérieure (25) fermée latéralement mais ouverte à au moins une extrémité longitudinale, l'étape b consistant à remplir ladite cavité intérieure (25) du substrat (20) avec au moins un matériau, notamment biocompatible.

15. Dispositif (120) d'exploration fonctionnelle intracérébrale, et/ou de stimulation et/ou de traitement par radio fréquence, multi-contact comportant au moins une sonde (100) d'exploration fonctionnelle intracérébrale multi-contact selon l'une quelconque des revendications 1 à 7 et au moins un appareil d'enregistrement et/ou de stimulation et/ou traitement (110) du patient connecté à ladite au moins une sonde (100).

## Patentansprüche

1. Mehrkontakt-Sonde (100) zur intrazerebralen funktionellen Untersuchung und/oder zur Stimulation und/oder zur Behandlung durch Hochfrequenz, umfassend:
einen distalen Teil (101) von zylindrischer Form, umfassend mindestens einen intrazerebralen Kontakt (11), der dazu bestimmt ist, in das Gehirn (131) eines Patienten implantiert zu werden,
einen proximalen Teil (103) von zylindrischer Form, umfassend mindestens einen Steckverbinderkontakt (77), der dazu bestimmt ist, an mindestens ein Aufzeichnungs- und/oder Stimulations- und/oder Behandlungsgerät (110) außerhalb des Körpers des Patienten angeschlossen zu werden, und
einen Verbindungsteil (102) von nicht zylindrischer Form, der den distalen Teil (101) und den proximalen Teil (103) verbindet,
wobei der distale Teil (101), der proximale Teil (103) und der Verbindungsteil (102) einen mehrschichtigen Film (5) beinhalten, der ein Substrat (20) und mindestens eine auf das Substrat (20) aufgebrachte leitfähige Schicht (10) umfasst, wobei das Substrat (20) mindestens ein Polymermaterial umfasst, wobei die mindestens eine leitfähige Schicht (10) mindestens eine Übertragungsbahn (12) und, in dem distalen Teil (101), den mindestens einen intrazerebralen Kontakt (11) sowie, in dem proximalen Teil (103), den mindestens einen Steckverbinderkontakt (77) umfasst, wobei jede Übertragungsbahn (12) mit einem intrazerebralen Kontakt (11) am distalen Teil (103) und mit einem Steckverbinderkontakt (77) am proximalen Teil (103) verbunden ist.

2. Sonde (100) nach Anspruch 1, wobei der mehrschichtige Film (5), auf das Substrat (20) aufgebracht, mindestens eine isolierende Schicht (30) aus einem Polymermaterial, bevorzugt aus einem Polymermaterial mit Flüssigkristallen, umfasst, wobei die mindestens eine isolierende Schicht (30) die mindestens eine Übertragungsbahn (12) mindestens teilweise bedeckt.

3. Sonde (100) nach einem der vorhergehenden Ansprüche, umfassend zwischen 1 und 60 intrazerebrale Kontakte (11), insbesondere zwischen 2 und 60 intrazerebrale Kontakte (11).

4. Sonde (100) nach einem der vorhergehenden Ansprüche, wobei das Substrat (20) aus mindestens einem Polymermaterial mit Flüssigkristallen (PCL) ausgeführt ist.

5. Sonde (100) nach einem der vorhergehenden Ansprüche, wobei der durch den Zylinder des distalen Teils (101) gebildete innere Hohlraum (25) mindestens teilweise mit mindestens einem Klebstoff oder einem Polymermaterial oder einem Verbundmaterial, insbesondere einem mit Metallpartikeln gefüllten Silikon, ausgefüllt ist.

6. Sonde (100) nach einem der vorhergehenden Ansprüche, wobei der distale Teil (101) mindestens einen Temperatursensor (35) umfasst, der insbesondere durch die mindestens eine leitfähige Schicht (10) gebildet wird.

7. Sonde (100) nach einem der vorhergehenden Ansprüche, wobei der distale Teil (101) ein distales Ende (71) umfasst, das durch ein distales Kontaktstück (70) verschlossen wird, und/oder wobei der distale Teil (101) ein proximales Ende (73) umfasst, das durch einen Stopfen (72) oder durch den mehrschichtigen Film (5) selbst verschlossen wird.

8. Verfahren zur Herstellung einer Mehrkontakt-Sonde (100) zur intrazerebralen funktionellen Untersuchung und/oder zur Stimulation und/oder zur Behandlung durch Hochfrequenz nach einem der vorhergehenden Ansprüche, wobei das Verfahren die folgenden Schritte umfasst:
a) Schritt a: Bilden eines mehrschichtigen Films (5), indem flach liegend auf mindestens einen Teil des Substrats (20) mindestens eine leitfähige Schicht (10), die mindestens einen intrazerebralen Kontakt (11) bildet, mindestens ein Steckverbinderkontakt (77) und mindestens eine Übertragungsbahn (12) aufgebracht werden, wobei jede Übertragungsbahn (12) mit einem intrazerebralen Kontakt (11) und mit einem Steckverbinderkontakt (77) verbunden ist,
b) Schritt b: Bilden eines Zylinders, der sich entlang einer Längsachse (Z) ausgehend von mindestens einem ersten Teil (21) des mehrschichtigen Films (5) erstreckt, der dazu bestimmt ist, den distalen Teil (101) der Sonde (100) zu bilden, die dazu bestimmt ist, in das Gehirn (131) eines Patienten implantiert zu werden, um Letztere zu erhalten,
c) Schritt c: Bilden eines Zylinders, der sich entlang einer Längsachse (Z) ausgehend von mindestens einem zweiten Teil (75) des mehrschichtigen Films (5) erstreckt, der dazu bestimmt ist, den proximalen Teil (101) der Sonde (100) zu bilden, die dazu bestimmt ist, an mindestens ein Aufzeichnungs- und/oder Stimulations- und/oder Behandlungsgerät (110) außerhalb des Patienten angeschlossen zu werden, um Letztere zu erhalten.

9. Verfahren nach dem vorhergehenden Anspruch, wobei der Schritt a, nach dem Aufbringen der mindestens einen leitfähigen Schicht (10) auf das Substrat (20), ein Komprimieren und/oder ein Erhitzen der mindestens einen leitfähigen Schicht (10) auf dem Substrat (20) umfasst, so dass die mindestens eine leitfähige Schicht (10) auf dem Substrat (20) fixiert wird.

10. Verfahren nach einem der Ansprüche 8 bis 9, wobei der erste Teil (21) und/oder der zweite Teil (75) des mehrschichtigen Films (5) zwei seitliche Ränder (22) umfasst, wobei der Schritt b und/oder der Schritt c ein mindestens teilweises Aufwickeln des ersten Teils (21) und/oder des zweiten Teils (75) des mehrschichtigen Films (5) umfasst, so dass die seitlichen Ränder (22) mindestens teilweise übereinander gelegt werden, wobei die seitlichen Ränder (22) bevorzugt durch Kleben und/oder Schweißen untereinander fixiert werden.

11. Verfahren nach einem der Ansprüche 8 bis 9, wobei der erste Teil (21) und/oder der zweite Teil (75) des mehrschichtigen Films (5) zwei seitliche Ränder (22) umfasst, wobei der Schritt b und/oder c ein mindestens teilweises Wickeln des ersten Teils (21) und/oder des zweiten Teils (75) des mehrschichtigen Films (5) umfasst, so dass die seitlichen Ränder (22) Rand an Rand in Kontakt gebracht werden.

12. Verfahren nach Anspruch 10 oder 11, wobei das Substrat (20) an mindestens einem der seitlichen Ränder (22) mindestens ein Fenster (23) umfasst, wobei der Schritt a so ausgeführt wird, dass die mindestens eine leitfähige Schicht (10) außerhalb des mindestens einen Fensters (23) aufgebracht wird.

13. Verfahren nach einem der Ansprüche 10 bis 12, wobei das Wickeln des ersten Teils (21) und/oder des zweiten Teils (75) des mehrschichtigen Films (5) ausgeführt wird, indem der erste Teil (21) und/oder der zweite Teil (75) des mehrschichtigen Films (5) in mindestens einen Kegelstumpf (40; 40a, 40b, 40c) sukzessive eingeführt wird, wobei der Durchmesser (dₑ) des äquivalenten kreisförmigen Querschnitts des ersten Teils (21) und/oder des zweiten Teils (75) des mehrschichtigen Films (5) nach dem sukzessiven Einführen in jeden Kegelstumpf (40; 40a, 40b, 40c) vermindert ist.

14. Verfahren nach einem der Ansprüche 8 und 9, wobei das Substrat (20) in seiner Dicke einen inneren Hohlraum (25) aufweist, der seitlich geschlossen ist, aber an mindestens einem Längsende offen ist, wobei der Schritt b darin besteht, den inneren Hohlraum (25) des Substrats (20) mit mindestens einem, insbesondere biokompatiblen, Material auszufüllen.

15. Mehrkontakt-Vorrichtung (120) zur intrazerebralen funktionellen Untersuchung und/oder zur Stimulation und/oder zur Behandlung durch Hochfrequenz, umfassend mindestens eine Mehrkontakt-Sonde (100) zur intrazerebralen funktionellen Untersuchung nach einem der Ansprüche 1 bis 7 und mindestens ein Aufzeichnungs- und/oder Stimulations- und/oder Behandlungsgerät (110) für den Patienten, das an die mindestens eine Sonde (100) angeschlossen ist.

## Claims

1. Multi-contact probe (100) for intracerebral functional investigation and/or stimulation and/or treatment by radiofrequencies, including:
• a distal part (101) of cylindrical shape including at least one intracerebral contact (11) intended to be implanted in the brain (131) of a patient,
• a proximal part (103) of cylindrical shape including at least one connector contact (77) intended to be connected to at least one device (110) for recording and/or stimulation and/or treatment outside the body of the patient, and
• a connecting part (102) of non-cylindrical shape connecting the distal part (101) and the proximal part (103),
the distal part (101), the proximal part (103) and the connecting part (102) comprising a multilayer film (5) including a substrate (20) and at least one conductive layer (10) deposited on the substrate (20), the substrate (20) including at least one polymer material, said at least one conductive layer (10) including at least one transmission track (12) and, in the distal part (101), said at least one intracerebral contact (11) as well as, in the proximal part (103), said at least one connector contact (77), each transmission track (12) being connected to an intracerebral contact (11) in the distal part (103) and to a connector contact (77) in the proximal part (103).

2. Probe (100) according to Claim 1, the multilayer film (5) including, deposited on the substrate (20), at least one insulative layer (30) of a polymer material, preferably a liquid crystal polymer material, said at least one insulative layer (30) at least partly covering said at least one transmission track (12).

3. Probe (100) according to either of the preceding claims, including between 1 and 60 intracerebral contacts (11), in particular between 2 and 60 intracerebral contacts (11).

4. Probe (100) according to any one of the preceding claims, the substrate (20) being made of at least one liquid crystal polymer (LCP) material.

5. Probe (100) according to any one of the preceding claims, the interior cavity (25) formed by the cylinder of the distal part (101) being at least partly filled with at least one glue or a polymer material or a composite material, in particular a silicone charged with metal particles.

6. Probe (100) according to any one of the preceding claims, the distal part (101) including at least one temperature sensor (35) formed in particular by said at least one conductive layer (10).

7. Probe (100) according to any one of the preceding claims, the distal part (101) including a distal end (71) closed by a distal stud (70) and/or the distal part (101) including a proximal end (73) closed by a plug (72) or by the multilayer film (5) itself.

8. Method of manufacturing a multi-contact probe (100) for intracerebral functional investigation and/or stimulation and/or treatment by radiofrequencies according to any one of the preceding claims, the method including the following steps:
a) Step a: forming a multilayer film (5) by depositing flat, on at least a part of the substrate (20), at least one conductive layer (10) forming at least one intracerebral contact (11), at least one connector contact (77) and at least one transmission track (12), each transmission track (12) being connected to an intracerebral contact (11) and to a connector contact (77),
b) Step b: forming a cylinder extending along a longitudinal axis (Z) from at least one first part (21) of the multilayer film (5) intended to form said distal part (101) of the probe (100) intended to be implanted in the brain (131) of a patient, in order to obtain the latter part,
c) Step c: forming a cylinder extending along a longitudinal axis (Z) from at least one second part (75) of the multilayer film (5) intended to form said proximal part (101) of the probe (100) intended to be connected to at least one device (110) for recording and/or stimulation and/or treatment outside the patient, in order to obtain the latter part.

9. Method according to the preceding claim, step a, after depositing said at least one conductive layer (10) on the substrate (20), including compression and/or heating of said at least one conductive layer (10) on the substrate (20) in such a manner as to fix said at least one conductive layer (10) on the substrate (20).

10. Method according to either of Claims 8 or 9, said first part (21) and/or said second part (75) of the multilayer film (5) including two lateral edges (22), step b and/or step c including at least partial rolling of said first part (21) and/or said second part (75) of the multilayer film (5) on itself or themselves in such a manner as at least partly to superpose said lateral edges (22), said lateral edges (22) preferably being glued and/or welded together.

11. Method according to either of Claims 8 or 9, said first part (21) and/or said second part (75) of the multilayer film (5) including two lateral edges (22), step b and/or c including at least partial rolling of said first part (21) and/or said second part (75) of the multilayer film (5) in such a manner as to bring said lateral edges (22) into edge-to-edge contact.

12. Method according to Claim 10 or 11, in which the substrate (20) includes on at least one of said lateral edges (22) at least one window (23), step a being performed in such a manner as to deposit said at least one conductive layer (10) outside said at least one window (23).

13. Method according to any one of Claims 10 to 12, in which said first part (21) and/or said second part (75) of the multilayer film (5) is or are rolled by successively inserting said first part (21) and/or said second part (75) of the multilayer film (5) into at least one truncated cone (40; 40a, 40b, 40c), the diameter (dₑ) of the equivalent circular section of said first part (21) and/or said second part (75) of the multilayer film (5) being reduced after successive insertion in each truncated cone (40; 40a, 40b, 40c).

14. Method according to either of Claims 8 or 9, in which the substrate (20) has within its thickness an interior cavity (25) closed laterally but open at one longitudinal end at least, step b consisting in filling said interior cavity (25) of the substrate (20) with at least one material, in particular at least one biocompatible material.

15. Multi-contact device (120) for intracerebral functional investigation and/or stimulation and/or treatment by radiofrequencies, including at least one multi-contact probe (100) for intracerebral functional investigation according to any one of Claims 1 to 7 and at least one device (110) for recording and/or stimulation and/or treatment of the patient connected to said at least one probe (100).
